(19) Europäisches Patentamt

European Patent Office

Office européen des brevets

(11) Veröffentlichungsnummer: **0 480 389 A1**

# (12) EUROPÄISCHE PATENTANMELDUNG

(21) Anmeldenummer: **91117169.2**

(22) Anmeldetag: **09.10.91**

(30) Priorität: **12.10.90 DE 4032354**

(43) Veröffentlichungstag der Anmeldung:
**15.04.92 Patentblatt 92/16**

(84) Benannte Vertragsstaaten:
**AT BE CH DE DK ES FR GB GR IT LI LU NL SE**

(51) Int. Cl.⁵: **C12P 21/00, A61K 37/02**

(71) Anmelder: **HOECHST AKTIENGESELLSCHAFT**
**Postfach 80 03 20**
**W-6230 Frankfurt am Main 80(DE)**

(72) Erfinder: **Schade, Ulrich F., Dr.**
**Gartenstrasse 20**
**W-2361 Wardersee(DE)**

(54) **Inhibitoren für die Bildung von Tumor Nekrose Fakfor, Verfahren zu deren Herstellung und deren Verwendung.**

(57) Es werden Inhibitoren für die Bildung von Tumor Nekrose Faktor, Verfahren zu deren Herstellung durch Makrophagen oder mononukleärer Phagozyten, die durch Lipopolysaccharide stimuliert wurden, und seine Verwendung zur Behandlung und Prophylaxe von durch Tumor Nekrose Faktor verursachter Krankheiten beschrieben.

Die Erfindung betrifft Inhibitoren für die Bildung von Tumor Nekrose Faktor, Verfahren zu deren Herstellung durch Makrophagen oder mononukleäre Phagozyten, die durch Lipopolysaccharide stimuliert wurden und deren Verwendung zur Behandlung und Prophylaxe von Krankheiten, die durch den Tumor Nekrose Faktor verursacht werden.

Entzündliche Reaktionen kommen in erster Linie dadurch zustande, daß immunologische Mechanismen, welche ursprünglich zum Schutze des Wirtsorganismus vorhanden sind, überexprimiert werden. Im Verlaufe der letzten Jahre ist deutlich geworden, daß Proteine, die vom Wirt gebildet werden, in isolierter Form Krankheitssymptome hervorrufen können, indem sie die Freisetzung terminaler entzündlicher Mediatoren bewirken. Diese Klasse von hormonartigen Proteinen wird unter dem Begriff Zytokine zusammengefaßt (Gupta, Scand J Rheumatol Suppl., (1988) 76, 189). Es gilt heute als gesichert, daß Zytokine für die Entstehung von entzündlichen Prozessen von grundlegender Bedeutung sind.

Unter den Zytokinen hat besonders der Tumor Nekrose Faktor (TNF) für die Entstehung akut inflammatorischer Prozesse Bedeutung erlangt, da diese im wesentlichen von Makrophagen gebildete Substanz in der Lage ist, in Säugetieren einen Schockstatus zu induzieren, wie er für Bakteriämien und Sepsis charakteristisch ist (Tracey et al. Science, 234, (1986), 470). Ferner wurde gefunden, daß ein monoklonaler Antikörper gegen TNF die Letalität in Affen während bakterieller Sepsis unterdrückt (Tracey et al., Nature, 330, (1987), 662). Aus diesen Befunden geht hervor, daß TNF einerseits ein ausreichender und andererseits ein essentieller Faktor für die Ausbildung vieler Charakteristika des endotoxischen Schocks ist.

Die offensichtliche Verknüpfung endogener TNF-Bildung mit der Entstehung akuter und chronisch entzündlicher Krankheitsbilder hat zu vielen Versuchen geführt die endogene Bildung von TNF durch pharmakologische Inhibitoren zu blockieren. Beispielhaft seien hier die Behandlung mit Steroiden, Xanthinderivaten und Lipoxygenasehemmstoffen genannt (Zabel et al., Lancet, 23, (1989), 1474). Ferner zeigte sich, daß das Zytokin IL 4 Inhibitor der TNF-Bildung ist (Essner et al., J. Immunol. 142, (1989), 3857).

Aufgabe der Erfindung war es, neue physiologische Inhibitoren zu finden, mit denen die endogene Bildung von TNF inhibiert werden kann.

Es wurde nun überraschenderweise gefunden, daß Makrophagen oder mononukleäre Phagozyten Inbitoren gegen die Bildung von TNF herstellen können.

Die Erfindung betrifft somit:

1. Inhibitoren gegen die Bildung von Tumor Nekrose Faktor, erhältlich durch Kultivierung von Makrophagen oder mononukleärer Phagozyten aus Lipopolysaccharid toleranten Wirbeltieren, nachfolgend TIM genannt.

2. Ein Verfahren zur Herstellung des unter 1. charakterisierten Inhibitors, das dadurch gekennzeichnet ist, daß Makrophagen oder mononukleäre Phagozyten aus Lipopolysaccharid-toleranten Wirbeltieren kultiviert werden und anschließend zu diesen Lipopolysaccharide hinzugefügt werden.

3. Die Verwendung des unter 1. charakterisierten Inhibitors zur Herstellung eines Medikaments zur Behandlung und Prophylaxe von durch Tumor Nekrose Faktor verursachten Erkrankungen.

Unter Lipopolysaccharid (LPS) werden Zellwandkomponenten von Gram negativen Bakterien verstanden. Sie bestehen aus einem Lipoid A und verschiedenen Polysacchariden, die je nach Bakterienart unterschiedliche Zuckerkomponenten enthalten können. LPS wirkt als Endotoxin, kann Fieber, Leukopenie und Leukocytose induzieren und bewirkt beim Kaninchen die lokale Schwarzmann-Reaktion. Beispielsweise kann aus Salmonella friedenau ein Lipopolysaccharid nach der Phenol-Wasser Methode extrahiert, lyophilisiert und in die Triethylamin Salzform überführt werden (Galanos et al., Zentralbl. Bakteriol. Parasitenkd. Infektionskd. Hyg. Abt. 1: Orig. Reihe A: 243, (1979), 226). Tumor Nekrosis Faktor (TNF) ist ein Zytokin, das vor allem von Makrophagen gebildet wird und lytisch auf bestimmt, transformierte Zellinien wirkt.

IL4 ist ein B-Zellen Wachstumsfaktor, der auf Makrophagen aktivierend wirkt und ein Molekulargewicht von 15 000 bis 20 000 hat. IL 6 ist ein B-Zellen Differenzierungsfaktor, der auf Hybridomazellinien als Wachstumsfaktor wirkt und ein Molekulargewicht zwischen 22 000 und 34 000 aufweist.

Der erfindungsgemäße Inhibitor von TNF ist ein Produkt, daß folgendermaßen charakterisiert werden kann:

- besitzt ein Molekulargewicht zwischen 40 000 und 80 000
- inhibiert die Synthese von TNF durch Makrophagen
- wirkt nicht wie Interleukin IL 6 als Wachstumsfaktor für Hybridomazellinien

Die Herstellung des Inhibitors erfolgt beispielsweise durch Makrophagen, die aus Lipopolysaccharid toleranten Wirbeltieren gewonnen werden können. Geeignet sind z.B. Makrophagen die aus Lipopolysaccharid-toleranten NMRI-Mäusen oder C3H/HeJ-Mäusen gewonnen werden (NMRI steht für Naval Marina Research Institut). Ferner können auch menschliche mononukleäre Phagocyten zur Herstellung von erfindungsgemäßen Inhibitoren verwendet werden.

Bei der Herstellung vom erfindungsgemäßen Inhibitor geht man am besten so vor, daß zuerst eine LPS-Toleranz in den Wirbeltieren induziert wird. So können beispielsweise weibliche NMRI-Mäuse (6 - 8 Wochen, Lippische Versuchstieranstalt, Dextertal, Deutschland) durch eine intraperitoneale Injektion von etwa 80 $\mu$g LPS, gewonnen von Salmonella friedenau nach der Phenol-Wasser-Methode, in 200 $\mu$l pyrogenfreiem Puffer tolerant gegen LPS gemacht werden. Nach 96 Stunden befinden sich die Mäuse im Zustand der LPS-Toleranz, der dadurch gekennzeichnet ist, daß bei Injektion von 2 mg LPS/Maus keines der toleranten Tiere stirbt. Bei normalen Mäusen entsprechen 0,7 mg des verwendeten LPS einer $LD_{100}$.

Aus den LPS-toleranten Mäusen werden nach literaturbekannten Methoden Peritoneal-Makrophagen gewonnen (Conrad, Manual of macrophages methodology. Ed. Herscowitz et al. (1981)) Die isolierten Makrophagen werden in sterile Kultivierungsgefäße überführt und anschließend in bekannten Kultivierungsmedien für Säugetierzellen, Zellinien und Gewebekulturen in einem $CO_2$-Brutschrank für 2 bis 3 Stunden inkubiert. Das Kultivierungsmedium wird abgenommen, nicht adhärente Zellen mit Puffer abgewaschen, frisches Medium zugegeben und mit LPS stimuliert.

Die nachfolgenden Mengenangaben für LPS beziehen sich auf das LPS von Salmonella friedenau, welches nach der Phenol-Wasser-Methode gewonnen wurde. Die Induktion der Makrophagen erfolgt mit etwa 1 bis 100 $\mu$g LPS/ml Medium, bevorzugt 5 bis 60 $\mu$g/ml. Die Makrophagen werden in einem $CO_2$-Brutschrank bei 37°C und 8 % $CO_2$ kultiviert. Die Makrophagen geben den erfindungsgemäßen Inhibitor in das umgebende Medium ab. Die maximale Inhibitorkonzentration wird 15 bis 36 Stunden nach Induktion erreicht. Die Makrophagen werden vom Medium abgetrennt, beispielsweise durch Zentrifugation oder Filtration. Die erfindungsgemäßen Inhibitoren verbleiben im Medium, aus dem sie durch gängige Methoden der Proteinanreicherung gewonnen werden können. Andere literaturbekannte Methoden der Kultivierung von Zellinien können ebenfalls verwendet werden. Es hat sich als vorteilhaft erwiesen die Makrophagen nach der ersten Synthese von TIM erneut mit LPS zu induzieren und mit frischem Kultivierungsmedium eine weitere TIM-Herstellung zu bewirken. So konnte die Herstellung von TIM über eine Woche beobachtet werden.

Ferner kann TIM auch durch Makrophagen von C3H/HeJ-Mäusen (Bomholtgård Ltd., Dänemark) hergestellt werden. Dabei entfällt die Behandlung der Mäuse durch LPS. Die Peritonealmakrophagen werden wie für die NMRI-Mäuse beschrieben isoliert, mit LPS induziert und TIM isoliert.

Eine weitere Methode zur Herstellung des erfindungsgemäßen Inhibitors besteht darin, ihn aus menschlichen mononukleären Phagozyten zu gewinnen. Dazu geht man am besten so vor, daß man zunächst an Probanden eine LPS-Toleranz erzeugt. Hierfür können beispielsweise 100 ng Salmonella abortus equi intravenös injiziert werden. 3 Tage nach Induktion werden 50 ml Blut entnommen aus denen nach literaturbekannten Methoden mononukleäre Phagozyten (Monozyten) gewonnen werden. Die Zellen können beispielsweise über einen ®Perkoll-Gradienten isoliert werden. Die isolierten Monozyten (1 x $10^6$ Zellen/ ml Medium) werden anschließend in literaturbekannten Kulturmedien für Säugetierzellen, Zellinien und Gewebekulturen mit üblichen Kultivierungsbedingungen für 2 bis 3 Stunden inkubiert. Die Induktion der Inhibitorsynthese erfolgt mit LPS. Dazu werden 1 bis 100 $\mu$g/ml Medium, bevorzugt 5 bis 60 $\mu$g/ml, in das Medium gegeben und die Monozyten werden unter den gleichen Kultivierungsbedingungen wie die Makrophagen aus LPS-toleranten Mäusen kultiviert und anschließend TIM isoliert.

Die charakteristische Wirkung des Inhibitors kann durch seine Wirkung auf die Synthese von TNF durch Makrophagen dargestellt werden. Die Wirkung der erfindungsgemäßen Inhibitoren kann beispielsweise durch die Zellinie RAW 264.7 (American Type Culture Collection 71 TIB (ATCC), Maryland, USA) bestimmt werden. Diese Zellinie bildet nach Stimulation durch LPS TNF. Die Bildung von TNF in dieser Zellinie kann durch TIM enthaltene Lösungen gehemmt werden.

Beispiel 1

Weibliche NMRI-Mäuse (6 - 8 Woche, Lippische Versuchstieranstalt, Dextertal, Deutschland) werden durch eine intraperitoneale Injektion von etwa 80 $\mu$g LPS, gewonnen aus Salmonella friedenau nach der Phenol-Wasser-Methode, in 200 $\mu$l pyrogenfreiem Puffer tolerant gegen LPS gemacht. Nach 96 Stunden befinden sich die Mäuse im Zustand der LPS-Toleranz, der dadurch gekennzeichnet ist, daß bei Injektion von 2 mg LPS/Maus keines der toleranten Tiere stirbt. Bei normalen Mäusen entsprechen 0,7 mg des verwendeten LPS einer $LD_{100}$. Aus den LPS-toleranten Mäusen werden die Peritoneal-Makrophagen gewonnen. Dazu werden die Mäuse durch $CO_2$-Einwirkung getötet, auf einer Korkplatte befestigt und mit 70 % Alkohol besprüht. In einer sterilen Werkbank wird dann das Fell durch einen Schnitt im Bauch- und Abdominalbereich eröffnet und 5 ml Ausspülmedium (Iscove's Medium + 2,5 U/ml Heparin; Sigma) in die Peritonealhöhle injiziert. Das so gefüllte Peritoneum wird vorsichtig massiert, um am Gewebe haftende Zellen abzulösen. Mittels einer sterilen Pasteurpipette wird die Zellen enthaltende Flüssigkeit abgesaugt.

Eine $10^7$ Zellen enthaltende Flüssigkeitsmenge wird in eine Zellkulturschale pipettiert und mit Kulturmedium (Iscove's Medium, 2 h, 37°C, 8 % $CO_2$) inkubiert. Um die Makrophagenkulturen von kontaminierenden Zelltypen zu reinigen, werden die Überstände von den adhärenten Zellen abgenommen, die Zellen zweimal mit je 10 ml Puffer (37°C) gewaschen, mit 10 ml frischem Iscove's Medium überschichtet. Dann wird 50 $\mu$g/ml LPS/ml Medium zugegeben.

Es wird LPS von Salmonella friedenau, isoliert nach der Phenol-Wasser-Methode, verwendet. Die Kultivierung der Makrophagen erfolgt in einem $CO_2$-Brutschrank bei 37°C und 8 % $CO_2$. Die Makrophagen geben den erfindungsgemäßen Inhibitor in das umgebende Medium ab. Die maximale Inhibitorkonzentration wird 15 bis 36 Stunden nach Induktion erreicht. Die Zellkulturüberstände werden zentrifugiert (1000 x g). Die Makrophagen sedimentieren und der Inhibitor verbleibt im Medium. Die Bestimmung der hergestellten TIM-Konzentration erfolgt durch die Wirkung von TIM auf die TNF-Herstellung der Zellinie RAW 264.7 (ATCC 71 TIB). Die in dem Medium enthaltene Inhibitormenge senkte die Synthese von TNF durch RAW 264.7 auf 30 % der Kontrolle.

TNF-Induktion: RAW 264.7-Zellen werden in Mikrotiterplatten gefüllt mit Iscove's Medium (Iscove Medium mit 10 % Fötalem Kälberserum (FCS); Gibco/BRL, Eggenstein, FRG) pipettiert. Es wird eine Zellkonzentration von etwa $1*10^6$ Zellen/ml Medium eingestellt. Nach 2 h werden die Zellen mit Puffer gewaschen, mit frischem Medium bedeckt und mit 200 ng LPS/ml in Gegenwart von TIM inkubiert. Es wird serumfreies Iscove's Medium verwendet. Nach 18 h Inkubationszeit werden die Überstände gewonnen und bei -70° bis zur TNF-Bestimmung gelagert.

Bestimmung von TNF: Die Bestimmung des TNF erfolgt im Bioassay mit der ATCC Fibroblasten Zellinie L929. Die Zellen werden in 100 $\mu$l Kulturmedium (RPMI 1640 + 10 % FCS + 5 $\mu$g/ml Actinomycin; Gibco) in 96 Loch-Platten (2 x $10^4$ Zellen/Loch; Flachboden, pipettiert. Die Zellen werden 4 h bei 37°C mit 5 % $CO_2$ in der Luft inkubiert. Dann werden je 100 $\mu$l der Verdünnungsreihen der zu testenden Proben auf die Zellen pipettiert. Diese Ansätze werden unter gleichen Bedingungen 18 h inkubiert und dann nach Zugabe von 10 $\mu$l/100 $\mu$l Kulturmedium einer Lösung von 5 mg/ml MTT (MTT = (3-(4,5-Dimethylthiazol-2-yl) -2,5-diphenyltetrazoliumbromid; gelöst in Puffer; Sigma, Deisenhofen) weitere 4 h inkubiert.

Danach werden die Überstände verworfen und durch 100 $\mu$l 0,04 N HCl in Isopronanol ersetzt. Nach intensivem Schütteln werden die Absorptionen bei 570 nm mit einem ELISA-Reader (MR 700, Dynatech Laboratories) gemessen. Die Berechnung des TNF-Titers der Probe erfolgte nach der folgenden Formel:

$$\% \text{ Zellzytotoxizität} = \frac{A_{con} - A_{dil}}{A_{con}} \times 100$$

$A_{con}$ ist die Absorption der Nullkontrolle. $A_{dil}$ ist die Absorption der Löcher der TNF-haltigen Verdünnungen. Eine Unit TNF ist definiert als der reziproke Wert derjenigen Verdünnung bei der 50 % Lyse erreicht werden.

Beispiel 2

Dosisabhängigkeit der Induktion

Peritoneal-Makrophagen werden aus LPS-toleranten Mäusen wie in Beispiel 1 beschrieben präpariert (1 x $10^6$ Zellen/ml) und in vitro mit unterschiedlichen LPS-Dosen inkubiert (18 hr, 37°C, 8 % $CO_2$). Aus den resultierenden Überständen wird die Inhibitor Aktivität bestimmt.

Die Makrophagen aus LPS-toleranten Mäusen benötigen in vitro eine Stimulation mit mindestens 50 $\mu$g/ml LPS um den erfindungsgemäßen Inhibitor (TIM) in guten Ausbeuten zu produzieren (Hemmung der TNF-Synthese um 45 ± 4,9 %, n = 5,p < 0,026 verglichen mit der LPS-Kontrolle). TIM gewonnen aus Makrophagenkulturen die mit 5 $\mu$g/ml LPS stimuliert wurden, erniedrigte die TNF-Freisetzung von 660 U/ml TNF auf 511 ± 43 U/ml ( = 78 ± 6 % der Kontrolle, n = 2). Die Kontrollen hatten eine Aktivität von 660 U/ml TNF.

Beispiel 3

Induktion der erfindungsgemäßen Inhibitoren in Makrophagen von C3H/HeJ Mäusen

Die LPS-low responder Mäuse des Stammes C3H/HeJ (Bomholtgard Ltd, Dänemark) zeichnen sich durch eine genetisch bedingte LPS-Toleranz aus. Peritoneal-Makrophagen wurden aus C3H/HeJ-Mäusen präpariert und mit verschiedenen LPS-Konzentrationen (0,5 $\mu$g/ml und 50 $\mu$g/ml) 18 hr in vitro kultiviert (1 x $10^6$/ml, 37°C, 8 % $CO_2$). Die Aufarbeitung erfolgte wie für die Makrophagen aus toleranten Mäusen beschrieben (Beispiel 1).

Nach der Aufarbeitung werden die rekonstituierten Lyophilisate auf Aktivität überprüft. Die TNF-Sekretion der mit LPS (200 $\mu$g/ml) stimulierten RAW 264.7-Zellen (Kontrolle) liegt bei 3050 U/ml TNF. Die Tabelle 1 zeigt, daß die C3H/HeJ-Mäuse eine geringe Menge an TIM herstellen. Durch in vitro Stimulation mit LPS kann die Bildung von TIM stark erhöht werden.

Tabelle 1

| Induktion der TNF-inhibitorischen Aktivität in Kulturüberständen von C3H/HeJ Mäusen | | | |
|---|---|---|---|
| Maus-Stamm | LPS ($\mu$g/ml) | Zellzahl (*$10^6$) | TNF-Titer im RAW 264.7-Assay (U/ml) |
| NMRI | 50 | 3,0 | 300 |
| NMRI | 50 | 1,0 | 100 |
| NMRI | 50 | 0,5 | 400 |
| C3H/HeJ | 0 | 3,0 | 2100 |
| C3H/HeJ | 0 | 1,0 | 2300 |
| C3H/HeJ | 0 | 0,5 | 2100 |
| C3H/HeJ | 5 | 3,0 | 800 |
| C3H/HeJ | 5 | 1,0 | 800 |
| C3H/HeJ | 5 | 0,5 | 1100 |
| C3H/HeJ | 50 | 3,0 | 335 |
| C3H/HeJ | 50 | 1,0 | 398 |
| C3H/HeJ | 50 | 0,5 | 335 |

Beispiel 4

TIM aus humanen peripheren Monozyten

Von zwei Endotoxin-behandelten Probanden (100 ng LPS von Salmonella abortus equi/Person) werden jeweils 3 Tage nach der Behandlung ein Volumen von 50 ml Blut abgenommen und daraus mononukleäre Phagozyten (Monozyten) präpariert. Diese werden durch Adhärenz gereinigt und mit LPS in serumfreiem Medium stimuliert (20 h, 50 $\mu$g/ml, 37°C, 8 % $CO_2$). Es wird aus den Überständen wie für die Mausmakrophagen beschrieben ein Rohextrakt hergestellt und auf TIM Aktivität getestet. Dies erfolgt in Kulturen von humanen Monozyten und in RAW-Zellen, die mit LPS zur Synthese von TNF angeregt werden. Wie aus Tabelle 2 hervorgeht, sind partiell gereinigte Überstände von LPS-stimulierten Monozyten aus humanen Probanden die mit Endotoxin behandelt werden (hTIM) in der Lage, die Synthese von TNF in Zellkulturen humaner Monozyten zu hemmen (hM$\phi$ + LPS: 870 ± 64 U/ml, hM$\phi$ + LPS + hTIM: 230 ± 42 U/ml). Wird muriner TIM (hergestellt wie in Beispiel 1 beschrieben) eingesetzt, so kann eine Hemmung der TNF-Synthese in demselben Ausmaß festgestellt werden (hM$\phi$ + LPS + mTIM: 190 ± 53 U/ml). Andererseits ist hTIM auch in der Lage in demselben Ausmaß TNF-Produktionen in mM$\phi$ zu blockieren wie mTIM (mM$\phi$ + LPS: 1026 ± 132 U/ml, mM$\phi$ + LPS + hTIM: 98 ± 24 U/ml, mM$\phi$ + LPS + mTIM: 134 ± 16 U/ml).

Daraus geht hervor, daß humane Monozyten von Endotoxin vorbehandelten Individuen befähigt sind, TIM zu synthetisieren und daß hTNF und mTNF in ihrer biologischen Wirksamkeit bezüglich der Inhibition der TNF-Bildung kreuzreagieren.

**Tabelle 2:   Wirkung humanen und murinen TIMs auf die TNF-Synthese humaner Monozyten und RAW 264.7 Zellen**

------------------------------------------------

| Inkubation | TNF (U/ml) |
|---|---|
| hMø | 44 ± 26 |
| hMø + LPS | 870 ± 64 |
| hMø + LPS + hTIM | 230 ± 42 |
| hMø + LPS + mTIM | 190 ± 53 |
| mMø | 56 ± 13 |
| mMø + LPS | 1026 ± 132 |
| mMø + LPS + hTIM | 98 ± 24 |
| mMø + LPS + mTIM | 134 ± 16 |

------------------------------------------------

Beispiel 5

Reinigung von TIM aus Peritoneal-Makrophagen von Mäusen

Durch Ultrafiltration kann der Inhibitor-haltige Überstand unter Erhalt der biologischen Aktivität konzentriert werden. Zellkulturüberstand toleranter, LPS-stimulierter Peritoneal-Makrophagen-Kulturen hergestellt nach Beispiel 1 ($1 \times 10^6$/ml, 50 $\mu$g/ml S.friedenau, 37°C, 8% $CO_2$) werden über eine Membran mit einer Ausschlußgrenze von 10 kD filtriert. Die Filtration wird beendet sobald das Probevolumen auf 10 % des Ausgangsvolumens eigeengt ist. Retentat und Filtrat werden sterilfiltriert und 100 $\mu$l Retentat bzw. 1 ml Filtrat werden auf TIM-Aktivität in RAW-Zellkulturen getestet. Die biologische Aktivität wird im Retentat wiedergefunden, d.h. diejenigen RAW 264.7-Zellen, deren Kulturmedium 10 % des aufkonzentrierten Zellkulturüberstandes enthält, sezernieren nach Stimulation mit LPS nur noch 17,8 ± 11,5 % (n = 6) LPS-stimulierter RAW Zellen, welche 1142 ± 87 U/ml TNF (n = 6) freisetzen. Der Inhibitor-haltige, konzentrierte Überstand enthält große Mengen an Lipopolysaccharid, welches durch Affinitätschromatographie an ®Polymyxin B-Agarose (Sigma) entfernt wird. 2 ml Polymyxin B-Agarose werden auf die Membran eines 0,2 $\mu$m Rundfilters aufgetragen, mit 15 ml pyrogenfreiem Wasser gewaschen und dann die LPS-haltigen Retentate mit einer Tropfgeschwindigkeit von etwa 1 ml/min chromatographiert. Das nach Polymyxin-Affinitätschromatographie erhaltene Material enthält Inhibitor-Aktivität und hemmt die TNF-Synthese von RAW Zellen um 71 % ($1 \times 10^6$ Zellen, 100 $\mu$l Eluat; 200 ng/ml S.friedenau, 18 h, 37°C, 8 % $CO_2$).

An die Chromatographie über Polymyxin B-Agarose schließt sich eine Dialyse gegen Wasser an, welche solange durchgeführt wird, bis kein Phenolrot, welches im Kulturmedium enthalten ist, sichtbar ist.

Danach wird das dialysierte Material gefriergetrocknet. Das lyophilisierte Material enthält die Inhibitor-Aktivität. Das in frischem Medium rekonstituierte Lyophilisat konnte die Produktion von TNF in RAW-Zellen auf 18 ± 6 % (n = 5) der LPS-stimulierten Kontrollen senken (p < 0,009).

Zur weiteren Reinigung des TIM enthaltenden Materials wird an ®Sephadex S-200 Gel chromatographiert (Phosphatpuffer 0,5 mmol + 0,2 mol NaCl, 3 ml/h).

Die biologische Aktivität wird im wesentlichen in Fraktion 6 gefunden: schon 100 $\mu$l (entsprechend 10 % des Gesamtvolumens) waren in der Lage, eine etwa 86 %ige Hemmung der TNF-Freisetzung zu bewirken (RAW 264.7, 1 x $10^6$ + 200 ng/ml LPS: 1149 U/ml TNF; RAW 264.7, 1 x $10^6$ + 200 ng/ml LPS + 100 $\mu$l TIM: 152 U/ml TNF). In der vergleichenden SDS-Gelelektrophorese ergibt sich ein Molekulargewicht von 40 000 bis 80 000.

Beispiel 6

Hemmung der Phorbolester-induzierten TNF-Synthese

Die TNF-Synthese wird durch eine Kombination aus Phorbolester (PMA) und Interferon (INF-$\gamma$ ; Amersham, Deutschland) induziert. RAW 264.7 Zellen (1 x $10^6$/ml) wurden mit LPS (10 $\mu$g/ml) und Interferon-$\gamma$ (50 U/ml) sowie verschiedenen Phorbolesterkonzentrationen (500 ng/ml, 100 ng/ml und 20 ng/ml) in Kombination mit Interferon-$\gamma$ (50 U/ml) inkubiert (18 h, 37°C, 8 % $CO_2$). Die RAW 264.7-Zellen produzieren sowohl nach Stimulation mit LPS/Interferon-$\gamma$ als auch Inkubation mit PMA / Interferon-$\gamma$ TNF (LPS/IFN-$\gamma$ : 2450 ± 274 U/ml TNF = 100 %, n = 4; 500 ng/ml PMA / 50 U/ml IFN-$\gamma$ : 1460 ± 60 U/ml TNF (n = 2), = 60 ± 2,5 % der durch LPS/IFN-$\gamma$ induzierbaren Menge). Die durch PMA/IFN-$\gamma$ induzierbare TNF-Menge ist dosisabhängig: durch 100 ng/ml PMA / 50 U/ml IFN-$\gamma$ wurden 1140 ± 213 U/ml (= 47 ± 8,6 % der LPS/IFN-$\gamma$ Kontrolle, n = 2) freigesetzt; 20 ng/ml PMA / 50 U/ml IFN-$\gamma$ führen zur Sekretion von 680 ± 164 U/ml TNF (= 28 ± 7 % der LPS/IFN-$\gamma$ Kontrolle).

Für die Untersuchung der Beeinflussung der PMA/IFN-induzierten TNF-Bildung durch TIM werden RAW Zellen mit PMA/IFN-$\gamma$ in Gegenwart vom Inhibitor inkubiert (1 x $10^6$/ml 18 H, 37°C, 8 % $CO_2$). Der erfindungsgemäße Inhibitor hemmt sowohl die LPS/IFN-$\gamma$ als auch die PMA/IFN-$\gamma$ induzierte TNF-Synthese und Freisetzung. Diejenige Inhibitormenge, welche die LPS/IFN-$\gamma$ induzierte TNF-Freisetzung um etwa 70 % auf 29 ± 6 % (n = 2; p < 0,08) der LPS-Kontrolle hemmt, inhibiert die PMA/IFN-$\gamma$ stimulierte TNF-Freisetzung um etwa 90 % auf 10 ± 4 % auf 40 U/ml TNF (n = 5, p < 0,04).

Beispiel 7

Inhibition der TNF-Synthese in Thioglycollat-induzierten Peritoneal-Makrophagen

Es werden Thioglycollat-induzierte Peritoneal-Makrophagen mit LPS und unterschiedlichen Mengen an Inhibitor 18 h inkubiert (1 x $10^6$/ml; 200 ng/ml LPS; 37°C, 8 % $CO_2$). Die eingesetzten Inhibitor-Mengen entsprechen den Mengen, die 1 x $10^7$/ml (= TIM 1), 5 x $10^6$/ml (= TIM 2), 2,5 x $10^6$/ml (= TIM 3) und 5 x $10^5$/ml (= TIM 4) Peritonealmakrophagen aus toleranten Mäusen sezernieren In diesen Versuchen werden auch in Kulturmedium rekonstituierte Lyophilisate von Überständen LPS-stimulierter Peritonealmakrophagen toleranter Mäuse verwendet. LPS induzierte in diesen Zellen TNF (6700 U/ml = 100 %). Unter Inhibitor-Einfluß sinkt die im Kulturüberstand nachweisbare TNF-Menge in Abhängigkeit von der eingesetzten Inhibitor-Menge: sowohl die Konzentration im Ansatz TIM 1 als auch TIM 2 war in der Lage, die TNF-Freisetzung auf < 40 U/ml (< 1 % der LPS-Kontrolle, n = 4; p < 0,04) zu supprimieren. Das Produkt von 2,5 x $10^6$ Peritoneal-Makrophagen bewirkt die Reduktion der TNF Bildung auf 1440 ± 100 U/ml TNF (entsprechend 21 ± 1,4 %, n = 2; p > 0,15); 5 x $10^5$ Peritonealmakrophagen-Produkt konnten die TNF-Freisetzung noch auf 34 ± 4,6 % erniedrigen (2260 ± 310 U/ml TNF; p > 0,22).

Beispiel 8

TIM hat keine IL 6-Aktivität

Ein Aliquot der die TIM-Aktivität enthaltenden Fraktion 6 nach Chromatographie an G 200 aus Beispiel 5 wird daraufhin untersucht, ob es die biologische Aktivität von Interleukin 6 (IL 6) enthält. Es wird ein Volumen eingesetzt, welches im RAW-Zellassay die Synthese von TNF um etwa 70 % hemmt. Dieses Volumen wird in einer seriellen Verdünnung (1:2) mit Zellen der Hybridoma-Zellinie B 13.29 (erhalten von Dr. L. Aarden, Central Laborastory Blood Transfusion Service, P. O. Box 9406, 1006 AK Amsterdam, Niederlande) in 96-Loch-Mikrotiterplatten zusätzlich zu 100 µl Zellkulturmedium (Iscove's Medium mit BSA, Transferrin, Sojabohnenlipide, $\beta$-Mercaptoethanol; Fertiglösungen der Firma Boeringer, Mannheim) für 48 h bei 37°C und 8 % $CO_2$ inkubiert. Als Kontrollwerte dienen B 13.29-Kulturen, die mit einer Konzentrationsreihe rekombinantem IL 6 (IC Chemikalien, München) behandelt werden. Die Anfärbung und Quantifizierung der Zellen geschieht wie in Beispiel 1. Durch Vergleich mit der Konzentrationsreihe IL 6 kann der absolute Gehalt an IL 6 bestimmt werden. Es zeigt sich, daß die B13.29-Zellen durch die die TIM-Aktivität enthaltende Fraktion des Makrophagenüberstandes nicht zum Wachstum angeregt wird, welches der Fall in den Kulturen ist, denen rekombinantes IL 6 zugesetzt wird. Daraus geht hervor, daß das getestete Aliquot kein IL 6 enthält und es sich bei TIM nicht um IL 6 handelt.

## Patentansprüche

1. Inhibitor gegen die Bildung von Tumor Nekrose Faktor, erhältlich durch Kultivierung von Makrophagen oder mononukleärer Phagozyten aus Lipopolysaccharid toleranten Wirbeltieren.

2. Inhibitor nach Anspruch 1, erhältlich durch Kultivierung von Makrophagen aus Lipopolysaccharid toleranten Mäusen.

3. Inhibitor nach Anspruch 1 oder 2, erhältlich durch Kultivierung von mononukleärer Phagozyten aus Lipopolysaccharid tolerenten Menschen.

4. Inhibitor nach einem oder mehreren der Ansprüche 1 bis 3, gekennzeichnet durch ein Molekulargewicht von 40 000 bis 80 000.

5. Verfahren zur Herstellung eines Inhibitors gemäß einem oder mehreren der Ansprüche 1 bis 4, dadurch gekennzeichnet, daß Makrophagen oder mononukleäre Phagozyten aus Lipopolysaccharid toleranten Wirbeltieren in Gegenwart von Lipopolysacchariden kultiviert werden.

6. Verfahren nach Anspruch 5, dadurch gekennzeichnet, daß Lipopolysaccharide von Salmonellen in einer Konzentration von 1 bis 100 µg je ml Kultivierungsmedium verwendet werden.

7. Verfahren nach Anspruch 6, dadurch gekennzeichnet, daß Lipopolysaccharide von Salmonellen in einer Konzentration von 5 bis 60 µg je ml Kultivierungsmedium verwendet werden.

8. Verfahren nach einem oder mehreren der Ansprüche 5 bis 7 dadurch gekennzeichnet, daß die Makrophagen oder mononukleären Phagozyten 10 Stunden bis 1 Woche, bevorzugt 15 bis 36 Stunden, kultiviert werden.

9. Verwendung eines Inhibitors gemäß einem oder mehreren der Ansprüche 1 bis 4 zur Herstellung eines Medikaments zur Behandlung oder Prophylaxe von durch Tumor Nektrose Faktor verursachten Erkrankungen.

### Patentansprüche für folgende Vertragsstaaten : ES, GR

1. Verfahren zur Herstellung eines Inhibitors gegen die Bildung von Tumor Nekrose Faktor dadurch gekennzeichnet, daß Makrophagen oder mononukleäre Phagozyten aus lipopolysaccharid toleranten Wirbeltieren in Gegenwart von Lipopolysacchariden kultiviert werden.

2. Verfahren nach Anspruch 1, dadurch gekennzeichnet, daß zur Kultivierung Makrophagen aus Lipopolysaccharid toleranten Mäusen verwendet werden.

3. Verfahren nach Anspruch 1 oder 2, dadurch gekennzeichnet, daß zur Kultivierung mononukleäre Phagozyten aus Lipopolysaccharid toleranten Menschen verwendet werden.

4. Verfahren nach einem oder mehreren der Ansprüche 1 bis 3, daurch gekennzeichnet, daß Lipopolysaccharide von Salmonellen in einer Konzentration von 1 bis 100 µg je ml Kultivierungsmedium verwendet werden.

5. Verfahren nach Anspruch 4, dadurch gekennzeichnet, daß Lipopolysaccharide von Salmonellen in einer Konzentration von 5 bis 60 $\mu$g je ml Kultivierungsmedium verwendet werden.

6. Vefahren nach einem oder mehreren der Ansprüche 1 bis 5, dadurch gekennzeichnet, daß die Makrophagen oder mononukleäre Phagozyten 10 Stunden bis 1 Woche, bevorzugt 15 bis 36 Stunden, kultiviert werden.

7. Verwendung eines Inhibitors gemäß einem oder mehreren der Ansprüche 1 bis 6 zur Herstellung eines Arzneimittels zur Behandlung oder Prophylaxe von durch Tumor Nekrose Faktor verursachten Erkrankungen.

| Kategorie | EINSCHLÄGIGE DOKUMENTE | | EP 91117169.2 |
|---|---|---|---|
| | Kennzeichnung des Dokuments mit Angabe, soweit erforderlich, der maßgeblichen Teile | Betrifft Anspruch | KLASSIFIKATION DER ANMELDUNG (Int CI.⁵) |
| P,A | EP - A - 0 422 339 (SYNERGEN) * Zusammenfassung * -- | 1 | C 12 P 21/00 A 61 K 37/02 |
| A | EP - A - 0 308 378 (YEDA RESEARCH AND DEVELOPMENT COMPANY LTD) * Pantentansprüche 1,27 * -- | 1,9 | |
| A | DE - A - 3 910 323 (GLAXO GROUP) * Patentansprüche * ---- | 1,9 | |

RECHERCHIERTE
SACHGEBIETE (Int. Cl.⁵)

C 12 P
A 61 K
C 12 N

Der vorliegende Recherchenbericht wurde für alle Patentansprüche erstellt.

| Recherchenort | Abschlußdatum der Recherche | Prüfer |
|---|---|---|
| WIEN | 20-12-1991 | WOLF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82